(19) Europäisches Patentamt
European Patent Office
Office européen des brevets

(11) Numéro de publication : **0 370 868 B1**

(12) # FASCICULE DE BREVET EUROPEEN

(45) Date de publication du fascicule du brevet : **03.03.93 Bulletin 93/09**

(51) Int. Cl.[5] : **A61K 7/48,** A61K 47/34, C08G 77/38

(21) Numéro de dépôt : **89403147.5**

(22) Date de dépôt : **16.11.89**

(54) **Utilisation de diorganopolysiloxanes modifiés comme anti-oxydants en cosmétique ou en dermatologie.**

(30) Priorité : **22.11.88 LU 87395**

(43) Date de publication de la demande : **30.05.90 Bulletin 90/22**

(45) Mention de la délivrance du brevet : **03.03.93 Bulletin 93/09**

(84) Etats contractants désignés : **AT BE CH DE ES FR GB IT LI NL SE**

(56) Documents cités :
**FR-A- 1 507 429**
**FR-A- 1 546 402**
**GB-A- 1 203 071**

(73) Titulaire : **L'OREAL**
**14, Rue Royale**
**F-75008 Paris (FR)**

(72) Inventeur : **Forestier, Serge**
**16, Allée Ferdinand Buisson**
**F-77410 Claye-Souilly (FR)**
Inventeur : **Lang, Gérard**
**44, Avenue Lacour**
**F-95210 Saint-Gratien (FR)**
Inventeur : **Richard, Hervé**
**48, Rue de l'Ermitage**
**F-75020 Paris (FR)**

(74) Mandataire : **Casalonga, Axel et al**
**BUREAU D.A. CASALONGA - JOSSE**
**Morassistrasse 8**
**W-8000 München 5 (DE)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne l'utilisation de diorganopolysiloxanes modifiés comme anti-oxydants dans des compositions cosmétiques ou dermatologiques ainsi que les compositions contenant ces composés.

Il est connu que les corps gras et certaines substances actives utilisés dans les compositions cosmétiques ou dermatologiques ont tendance à s'oxyder, même à température ambiante.

Cette oxydation leur fait acquérir de nouvelles propriétés, notamment olfactives, qui sont indésirables lorsque ces substances sont incorporées dans des compositions cosmétiques ou dermatologiques.

Pour éviter cette oxydation, on utilise en général des agents protecteurs jouant le rôle d'anti-oxydants. On utilise couramment le ditertiobutylhydroxytoluène. Cependant, ce composé présente une solubilité limitée dans certains corps gras.

La demanderesse a découvert que certains diorganopolysiloxanes porteurs de motifs alkylphénoliques, possédaient une solubilité sensiblement améliorée dans les corps gras et présentaient simultanément d'excellentes propriétés antioxydantes vis-à-vis de la peroxydation de lipides polyinsaturés et également vis-à-vis des substances susceptibles de subir des réactions d'oxydation thermo-ou photo-induites (telles que des protéines, des sucres, des pigments, des vitamines, des polymères).

La demanderesse a découvert, d'une manière surprenante, que les diorganopolysiloxanes conformes à la présente invention permettaient d'assurer une meilleure conservation des compositions cosmétiques ou dermatologiques comportant une phase grasse en évitant le rancissement de lipides insaturés qui y sont contenus, qu'ils pouvaient également permettre d'éviter la dégradation oxydative de composés actifs contenus dans ces compositions, telles que la vitamine A ou les caroténoïdes.

Les polymères conformes à la présente invention présentent, en outre, l'avantage de ne pas être absorbés par la peau.

La présente invention a pour objet l'utilisation de diorganopolysiloxanes à motifs alkylphénoliques comme agents antioxydants dans des compositions cosmétiques ou dermatologiques.

Un autre objet de l'invention consiste en des compositions cosmétiques ou dermatologiques, contenant les diorganopolysiloxanes à motifs alkylphénoliques définis ci-après.

D'autres objets conformes à l'invention apparaîtront à la lecture de la description.

Les diorganopolysiloxanes, utilisés comme antioxydants, conformément à la présente invention, sont choisis :

(i) parmi ceux de formule (I) :

$$R_2\underset{B}{\underset{|}{Si}}O\,(R_2SiO)_a\,(R\underset{A}{\underset{|}{Si}}O)_b\,\underset{B}{\underset{|}{Si}}R_2 \qquad (I)$$

dans laquelle, les symboles :

R, identiques ou différents, sont choisis parmi les radicaux alkyles en $C_1$-$C_{10}$ ou phényle, au moins 80% en nombre des radicaux R étant méthyle,

B, identiques ou différents, sont choisis parmi les radicaux R et le radical A,

a est un nombre entier choisi entre 0 et 200 inclus,

b est un nombre entier choisi entre 0 et 50 inclus, et si b est égal à 0, au moins un des deux symboles B est A,

A est un radical de formule :

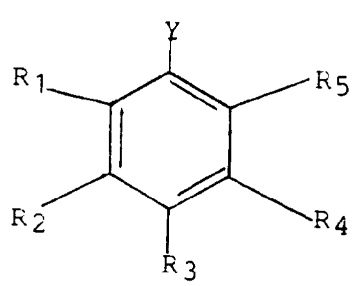

(III)

dans laquelle :

$R_1$ représente un atome d'hydrogène, un radical alkyle linéaire ou ramifié en $C_1$-$C_8$,

$R_2$ représente un atome d'hydrogène, un radical hydroxyle, un radical alkyle linéaire ou ramifié en $C_1$-$C_8$, ou un radical alcoxy linéaire ou ramifié en $C_1$-$C_8$,

$R_3$ représente un atome d'hydrogène, un radical hydroxyle ou un radical alkyle linéaire ou ramifié en $C_1$-$C_8$,

$R_4$ représente un atome d'hydrogène ou un radical alkyle linéaire ou ramifié en $C_1$-$C_8$,

$R_5$ représente un atome d'hydrogène, un radical hydroxyle ou un radical alcoxy linéaire ou ramifié en $C_1$-$C_8$,

étant entendu que l'un au moins des radicaux $R_2$, $R_3$ ou $R_5$ représente un radical hydroxyle, et que lorsque $R_2$ est alcoxy et $R_3$ est hydroxy, l'un au moins des radicaux $R_1$, $R_4$ ou $R_5$ est différent d'un atome d'hydrogène,

Y représente un radical divalent :

$$-(O)_n-CH_2-CH(R_6)-CH_2-$$

dans lequel :

$R_6$ représente un atome d'hydrogène ou un radical alkyle en $C_1$-$C_4$ et n vaut 0 ou 1,

(ii) et ceux de formule (II) :

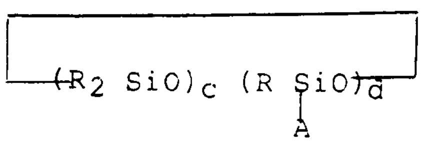

(II)

dans laquelle :

R et A ont les mêmes significations que celles indiquées dans la formule (I),

c est un nombre entier compris entre 1 et 20 inclus,

d est un nombre entier compris entre 2 et 20 inclus,

c + d est égal ou supérieur à 3.

Parmi les composés particulièrement préférés utilisés conformément à l'invention, on peut citer les diorganopolysiloxanes statistiques ou à blocs de formules (I) ou (II), dans lesquelles :

- R est méthyle,
- B est méthyle,
- a est compris entre 5 et 20 inclus,
- b est compris entre 2 et 15 inclus,
- c + d est compris entre 3 et 10 inclus,
- dans le radical A, les radicaux alkyle sont choisis parmi les radicaux méthyle, éthyle, n-propyle, n-butyle, tert-butyle, tétraméthyl-1,1,3,3 butyle, les radicaux alcoxy sont de préférence des méthoxy, et Y désigne un radical $-(CH_2)_3-$ ou $-O(CH_2)_3-$.

Les diorganopolysiloxanes de formules (I) ou (II), sont obtenus en faisant réagir un composé de formule :

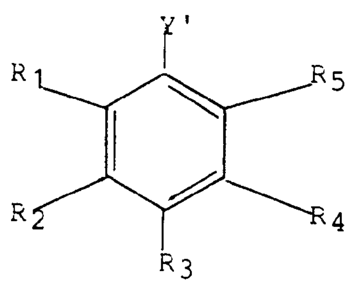

(VI)

dans laquelle $R_1$, $R_2$, $R_3$, $R_4$ et $R_5$ ont la même signification qu'indiquée dans la formule (III) ci-dessus et Y' est le radical insaturé de formule :

$$-(O)_n-CH_2-C(R_6)=CH_2$$

dans laquelle n et $R_6$ ont la même signification qu'à la formule (III),

sur un copoly(organohydro-diorgano)siloxane, de formule :

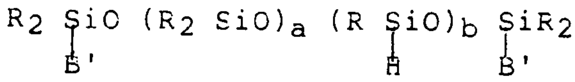

(IV)

dans laquelle R, a et b ont la signification indiquée ci-dessus dans la formule (I) et les radicaux B′, identiques ou différents, sont choisis parmi les radicaux R et un atome d'hydrogène, et ceux de formule :

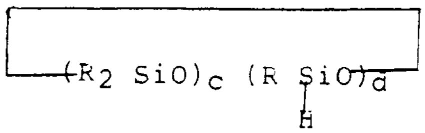

(V)

dans laquelle R, c et d ont la signification indiquée ci-dessus dans la formule (II).

Cette réaction sera dénommée par la suite réaction d'hydrosilylation.

La réaction d'hydrosilylation est effectuée en présence d'un catalyseur à base de platine, tel que le platine sur charbon, l'acide hexachloroplatinique, le produit de réaction de l'acide hexachloroplatinique avec un alcool, un aldéhyde ou un éther, l'iodure de triméthylplatine, l'hexaméthyldiplatine, le platine complexé avec des dérivés possédant des insaturations, par exemple des oléfines ou des vinyl-siloxanes. Ces catalyseurs sont décrits dans les brevets US-A-2.823.218, 3.220.972, 3.313.773, 3.159.601 et 3.159.662.

La réaction d'hydrosilylation peut être effectuée, éventuellement, en présence d'un solvant volatil inerte, à une température comprise entre la température ambiante et 200°C, selon la nature du catalyseur. La concentration en catalyseur est comprise entre $10^{-7}$ et $10^{-3}$ et de préférence entre $10^{-5}$ et $10^{-4}$ atome-gramme de platine par mole de composé de formule (VI). Le composé de formule (VI) est ajouté lentement au copoly(organohydro-diorgano)siloxane de formule (IV) ou (V) contenant la quantité nécessaire de catalyseur, à une vitesse suffisante pour maintenir le mélange réactionnel à une température généralement comprise entre 50 et 120°C.

On peut également ajouter simultanément le composé de formule (VI) et le copoly(organohydro-diorgano)siloxane de formule (IV) ou (V) à une suspension de catalyseur dans un solvant ou bien ajouter le copoly(organohydro-diorgano)siloxane de formule (IV) ou (V) au composé de formule (VI), éventuellement en solution dans un solvant, et contenant la quantité nécessaire de catalyseur. La réaction est effectuée en utilisant des proportions stoechiométriques des réactifs (IV), (V) et (VI) ou éventuellement un léger défaut du réactif (VI) ou du copoly(organohydro-diorgano)siloxane de formule (IV) ou (V).

Parmi les dérivés de formule (VI) définis ci-dessus, on peut citer plus particulièrement :

- l'allyl-4 ditert-butyl-2,6 phénol,
- l'allyl-2 ditert-butyl-4,6 phénol,
- l'allyl-2 méthoxy-4 tert-butyl-6 phénol,
- l'allyl-2 méthyl-4 tert-butyl-6 phénol,
- l'allyl-2 méthyl-4(tétraméthyl-1,1,3,3 butyl)-6 phénol,
- l'allyloxy-4 ditert-butyl-2,6 phénol,
- l'allyloxy-4 tert-butyl-2 phénol,
- l'allyl-2 tert-butyl-5 hydroquinone.

Les dérivés de formule (VI) sont connus et peuvent être préparés selon des méthodes connues.

A titre d'exemple, les dérivés de formule (VI) dans laquelle Y′ représente un radical insaturé de formule $(O)_n-CH_2-C(R_6)=CH_2$, lorsque n=1, peuvent être obtenus par réaction d'un halogénure d'alcényle de formule (VII) sur un dérivé de formule (VIII) :

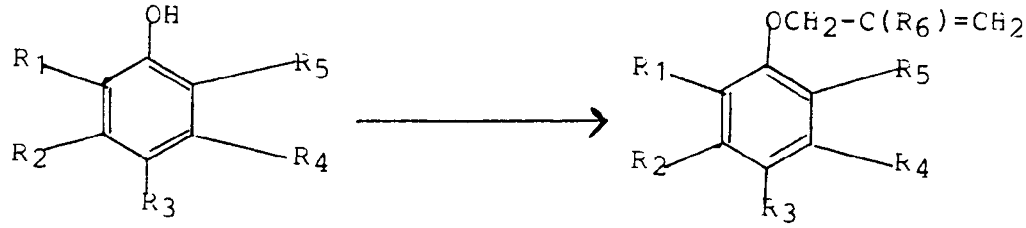

$X-CH_2-C(R_6)=CH_2$

(VIII) (VII) (VIA)

Cette réaction est effectuée en présence d'une base dans un solvant, ou un mélange de solvants, par exemple en présence d'un carbonate de métal alcalin dans le diméthylformamide ou en présence d'un hydroxyde de métal alcalin et d'un catalyseur de transfert de phase dans un mélange de toluène et d'eau, à une température comprise entre la température ambiante et le point d'ébullition du solvant.

Dans les composés de formule (VIA), (VII) et (VIII), $R_1$, $R_2$, $R_3$, $R_4$, $R_5$, $R_6$ ont les significations indiquées ci-dessus et X représente un atome d'halogène, de préférence un atome de chlore ou de brome.

Les dérivés de formule (VI) dans laquelle $R_6$=OH et Y′ représente un radical insaturé de formule $-(O)_n-CH_2-C(R_6)=CH_2$, lorsque n=0, peuvent être obtenus par réarrangement de CLAISEN d'un composé de formule (IX) selon le schéma réactionnel ci-dessous :

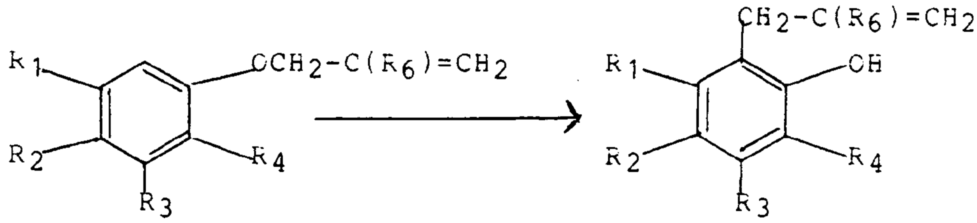

(IX) (VIB)

Ce réarrangement peut être effectué dans les conditions décrites par TARBELL (Organic réactions, vol. 2, John Wiley, New York, 1944, page 1) par chauffage à au moins 170°C environ du composé de formule (IX) éventuellement en présence d'un solvant.

Le composé de formule (IX) peut être obtenu par réaction d'un halogénure d'alcényle de formule (VII) sur un composé de formule (X) :

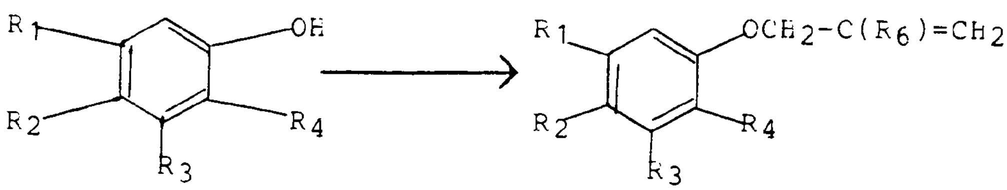

$X-CH_2-C(R_6)=CH_2$

(X) (VII) (IX)

Cette réaction est effectuée en présence d'une base dans un solvant ou un mélange de solvants, par exemple en présence d'un carbonate de métal alcalin dans le diméthylformamide ou en présence d'un hydroxyde de métal alcalin et d'un catalyseur de transfert de phase dans un mélange de toluène et d'eau, à une température comprise entre la température ambiante et le point d'ébullition du solvant. Le composé de formule (X) peut être préparé selon des méthodes connues. Dans les composés de formules (VIB), (VII), (IX) et (X), $R_1$, $R_2$, $R_3$, $R_4$, $R_6$ et X ont les significations indiquées ci-dessus.

Les dérivés de formule (VI) dans laquelle $R_3$=OH, $R_1$=$R_5$=H, $R_2$ et $R_4$ représentent un radical alkyle linéaire ou ramifié en $C_1$-$C_8$ et Y′ représente un radical insaturé de formule $-(O)_n-CH_2-C(R_6)=CH_2$, lorsque n=0, peuvent être obtenus par réaction d'un halogénure d'alcényle de formule (VII) sur un dérivé de formule (XI) :

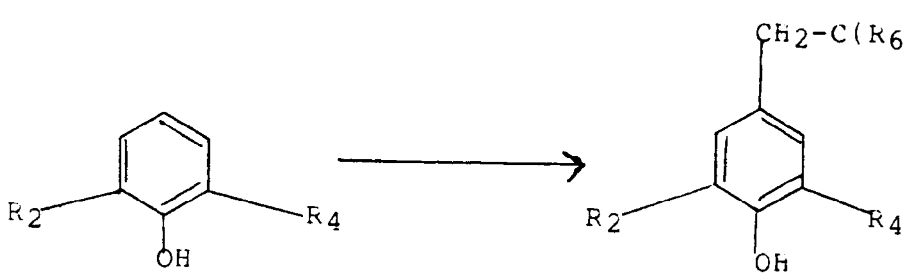

$X-CH_2-C(R_6)=CH_2$

(XI) (VII) (VIC)

Cette réaction est effectuée en présence d'une base dans un solvant, ou un mélange de solvants, par exemple en présence d'un alcoolate ou d'un carbonate de métal alcalin dans le diméthylformamide ou en présence d'un hydroxyde de métal alcalin éventuellement en présence d'un catalyseur de transfert de phase dans le toluène, à une température comprise entre la température ambiante et le point d'ébullition du solvant. Les composés de formule (XI) sont des composés connus.

Les compositions cosmétiques ou dermatologiques, conformes à l'invention, sont caractérisées essentiellement par le fait qu'elles contiennent dans un milieu cosmétiquement ou dermatologiquement acceptable, contenant une phase grasse d'origine animale, végétale ou synthétique et/ou une substance active susceptible de s'oxyder, au moins un diorganopolysiloxane de formule (I) ou (II) dans des concentrations comprises entre 0,01 et 5%, et de préférence entre 0,05 et 3% en poids par rapport au poids total de la composition.

Les corps gras utilisés conformément à la présente invention sont constitués de lipides insaturés d'origine animale, tels que la lanoline, la cétine (blanc de baleine), la cire d'abeille, le perhydrosqualène, l'huile de tortue, ou végétale comme l'huile d'olive, l'huile de ricin, l'huile d'avocat, l'huile de tournesol, l'huile de soja, l'huile d'arachide, l'huile de maïs, l'huile de karité, l'huile d'amandes douces, l'huile de sésame, l'huile de cassis, les huiles de coprah ou de palmiste, les graisses comme le beurre de cacao, les cires telles que la cire de Carnauba, la cire de Montana, la cire de Candellila, les acides gras essentiels comme la vitamine F et les huiles essentielles présentes dans les parfums, comme l'huile de citron ou de lavande.

Les substances actives utilisés conformément à la présente invention sont choisies parmi celles utilisées généralement dans le domaine de la cosmétique ou de la dermatologie, telles que la vitamine A, les caroténoïdes, les protéines, les pigments naturels, les sucres, les polymères.

Ces compositions peuvent également contenir des épaississants, des solvants compatibles avec la peau, tels que les alcools inférieurs en $C_1$-$C_4$ comme l'éthanol, des polyalcools, tels que le propylèneglycol, ou tout autre solvant approprié et des adoucissants, des surgraissants, des émollients, des mouillants, des tensio-actifs anioniques, cationiques, non ioniques, amphotères ou leurs mélanges, des filtres solaires, des colorants ayant pour fonction de colorer la peau ou la composition elle-même, et tout autre ingrédient habituellement utilisé dans des compositions cosmétiques ou dermatologiques.

Les compositions cosmétiques ou dermatologiques conformes à la présente invention se présentent sous la forme de solutions huileuses, d'émulsions huile-dans-eau ou eau-dans-huile (crèmes ou laits), de bâtonnets solides, de lotions plus ou moins épaissies, de gels, de pommades, de tampons imbibés, de sprays aérosol ou mousse, ou encore de pains de savon.

Une forme de réalisation préférée de l'invention est constituée par des lotions, des crèmes et des laits pour le soin du visage, des laits et des crèmes corporels, des laits ou des crèmes démaquillants, des fonds de teint, des crèmes teintées, des bases de maquillage, des masques, des rouges à lèvres, des fards à paupières, etc.

Un autre objet de l'invention consiste en un procédé de protection vis-à-vis de l'oxydation de substances cosmétiques ou dermatologiques oxydables, caractérisé par le fait que l'on incorpore auxdites substances une quantité efficace d'au moins un diorganopolysiloxane de formule (I) ou (II).

Les exemples qui suivent servent à illustrer l'invention.

<u>EXEMPLE 1</u>

Préparation d'un composé de formule générale (I) dans laquelle R = B = $CH_3$, a = b = 6, et A représente un radical de formule (III) dans laquelle $R_1$ = $R_5$ = H, $R_2$ = $R_4$ = tert-butyl, $R_3$ = OH et Y = $-(CH_2)_3-$.

A une suspension de platine sur charbon à 5% (520 mg) dans du toluène sec (25 ml) à 90-100°C, sous azote et sous agitation, on ajoute goutte à goutte en une heure 45 minutes, une solution toluénique (175 ml) d'allyl-4 ditert-butyl-2,6 phénol (90 g, 365 meq) et de polyméthylhydro-(45-50%)diméthylsiloxane copolymer (Petrarch Systems Inc., PS 122.5, 54 g, 332 meq en SiH) tout en maintenant la température entre 100 et 105°C. On laisse sous agitation et au reflux jusqu'à disparition des groupements SiH (absence de bande à 2180 $cm^{-1}$ en infrarouge), soit 8 heures. On filtre sur papier, rince le solide avec du dichlorométhane et élimine les solvants. L'huile brun rouge obtenue (143,6 g) est chromatographiée sur gel de silice 60. Par élution avec un mélange dichlorométhane/heptane 20/80, on élimine l'excès d'oléfine. L'élution avec du dichlorométhane pur permet d'obtenir le produit attendu sous forme d'une huile épaisse jaune pâle (106 g, rendement = 78%).

Spectre $^1$H RMN ($CDCl_3$) : spectre conforme à la formule
Spectre $^{29}$Si RMN ($CDCl_3$): spectre conforme à la formule

<u>EXEMPLE 2</u>

Préparation d'un composé de formule générale (I) dans laquelle R =B = $CH_3$, a = b = 6, et A représente un

radical de formule (III) dans laquelle $R_1 = R_3 = H$, $R_2 = R_4$ = tert-butyl, $R_5$ = OH et Y = $-(CH_2)_3-$.

1) Préparation du ditert-butyl-2,4 allyloxy benzène.

Dans un réacteur, on ajoute successivement de l'hydroxyde de sodium en pastilles (20 g, 0,5 mole), de l'eau (25 g), du toluène (50 ml) et de l'hydrogénosulfate de tétrabutylammonium (1 g). On porte à 50°C sous agitation et sous azote et ajoute en 45 minutes une solution toluénique (50 ml) de bromure d'allyle (16,5 g, 0,134 mole) et de ditert-butyl-2,4 phénol (25 g, 0,12 mole). La température monte progressivement jusqu'à 68°C. On laisse à cette température pendant 3 heures. On refroidit, ajoute 50 ml d'eau et 50 ml de toluène et décante. La phase toluénique est lavée à l'eau, séchée sur sulfate de sodium et le solvant est évaporé pour donner une huile orangée qui est purifiée sur un lit de gel de silice 60 (éluant : heptane). On obtient le ditert-butyl-2,4 allyloxy benzène sous forme d'un liquide incolore (23 g, rendement = 78%).

2) Préparation de l'allyl-2 ditert-butyl-4,6 phénol.

10 g de ditert-butyl-2,4 allyloxy benzène sont chauffés sous azote à 245°C pendant 45 minutes. L'huile brute jaune est reprise dans de l'éther diisopropylique. La phase éthérée est lavée à la soude à 3% puis à l'eau. Après séchage et évaporation du solvant, l'huile obtenue (7,6 g ) est chromatographiée sur gel de silice 60 (éluant : heptane/$CH_2Cl_2$:80/20). On obtient une huile jaune pâle d'allyl-2 ditert-butyl-4,6 phénol (5,4 g, rendement = 54%).

3) Réaction d'hydrosilylation.

A une suspension de platine sur charbon à 5% (27 mg) dans du toluène sec (5 ml) à 90-100°C, sous azote et sous agitation, on ajoute goutte à goutte en 45 minutes, une solution toluénique (10 ml) d'allyl-2 ditert-butyl-4,6 phénol (4,3 g, 17,5 meq) et de polyméthylhydro(45-50%)diméthylsiloxane copolymer (Petrarch Systems Inc., PS 122,5, 2,56 g, 16 meq en SiH) tout en maintenant la température entre 100 et 105°C. On laisse sous agitation et au reflux jusqu'à disparition des groupements SiH (absence de bande à 2180 $cm^{-1}$ en infrarouge), soit 8 heures. On filtre sur papier et on élimine le solvant. L'huile jaune orangée obtenue (6,8 g) est chromatographiée sur gel de silice 60. Par élution avec un mélange dichlorométhane/heptane 35/65, on élimine l'excès d'oléfine. L'élution avec du dichlorométhane pur permet ensuite d'obtenir, après évaporation du solvant, une huile épaisse jaune pâle (2,9 g, rendement = 45%).

Spectre $^{1}H$ RMN($CDCl_3$): spectre conforme à la formule.
Spectre $^{29}Si$ RMN($CDCl_3$): spectre conforme à la formule.

EXEMPLE 3

Préparation d'un composé de formule générale (I) dans laquelle R = B = $CH_3$, a = b = 6, et A représente un radical de formule (III) dans laquelle $R_1 = R_3 = H$, $R_2 = OCH_3$, $R_4$ = tert-butyl, $R_5$ = OH et Y = $-(CH_2)_3-$.

A une suspension de platine sur charbon à 5% (50 mg) dans du toluène sec (5 ml) à 90-100°C, sous azote et sous agitation, on ajoute goutte à goutte en 90 minutes, une solution toluénique (30 ml) d'allyl-2 méthoxy-4 tert-butyl-6 phénol (8,6 g, 39 meq) et de polyméthylhydro-(45-50%)diméthylsiloxane copolymer (Petrarch Systems Inc., PS 122,5, 5,77 g, 35,5 meq en SiH) tout en maintenant la température entre 100 et 105°C. On laisse sous agitation et au reflux jusqu'à disparition des groupements SiH (absence de bande à 2180 $cm^{-1}$ en infrarouge), soit 8 heures. On filtre sur papier, élimine le solvant et obtient une huile orangée que l'on chromatographie sur gel de silice 60. L'oléfine de départ est récupérée en utilisant comme éluant un mélange dichlorométhane/heptane 20/80. Par élution au dichlorométhane pur on obtient, après évaporation du solvant, le produit attendu sous forme d'une huile épaisse orangée (12 g, rendement = 88%).

Spectre $^{1}H$ RMN ($CDCl_3$) : spectre conforme à la formule
Spectre $^{29}Si$ RMN($CDCl_3$): spectre conforme à la formule.

EXEMPLE 4

Préparation d'un composé de formule générale (I) dans laquelle R = B = $CH_3$, a = b = 6, et A représente un radical de formule (III) dans laquelle $R_1 = R_3 = H$, $R_2$ = méthyle, $R_4$ = tert-butyle, $R_5$ = OH et Y = $-(CH_2)_3-$.

A une suspension de platine sur charbon à 5% (101 mg) dans du toluène sec (5 ml) à 90-100°C, sous azote et sous agitation, on ajoute goutte à goutte en 90 minutes, une solution toluénique (60 ml) d'allyl-2 méthyl-4 tert-butyl-6 phénol (16,5 g, 81 meq) et de polyméthylhydro-(45-50%)diméthylsiloxane copolymer (Petrarch

Systems Inc., PS 122,5, 12,47 g, 76,8 meq en SiH) tout en maintenant la température entre 100 et 105°C. On laisse sous agitation et au reflux jusqu'à disparition des groupements SiH (absence de bande à 2180 $cm^{-1}$ en infrarouge), soit 8 heures. On filtre sur papier, élimine le solvant et lave deux fois à l'éthanol à 70%. L'huile jaune orangée obtenue est reprise dans le dichlorométhane. La phase organique est séchée sur sulfate de sodium et filtrée sur lit de gel de silice 60. On obtient, après évaporation du solvant, le produit attendu sous forme d'une huile épaisse jaune-orangée (26 g, rendement = 92%).

Spectre $^1H$ RMN ($CDCl_3$) : spectre conforme à la formule
Spectre $^{29}Si$ RMN ($CDCl_3$): spectre conforme à la formule.

EXEMPLE 5

Préparation d'un composé de formule générale (I) dans laquelle R = B = $CH_3$, a = b = 6, et A représente un radical de formule (III) dans laquelle $R_1$ = $R_2$ = $R_5$ = H, $R_4$ = tert-butyl, $R_3$ = OH et Y = $-O-(CH_2)_3-$.

A une suspension de platine sur charbon à 5% (15 mg) dans du toluène sec (5 ml) à 90-100°C, sous azote et sous agitation, on ajoute goutte à goutte en trente minutes, une solution toluénique (15 ml) d'allyloxy-4 tert-butyl-2 phénol (2,1 g, 10 meq) et de polyméthylhydro-(45-50%)diméthylsiloxane copolymer (Petrarch Systems Inc., PS 122,5, 1,49 g, 9,2 meq en SiH) tout en maintenant la température entre 100 et 105°C. On laisse sous agitation et au reflux jusqu'à disparition des groupements SiH (absence de bande à 2180 $cm^{-1}$ en infrarouge), soit 8 heures. On filtre sur papier, élimine le solvant et lave deux fois à l'éthanol à 70%. L'huile jaune pâle obtenue est reprise dans le chloroforme. La phase organique est séchée sur sulfate de sodium et filtrée sur lit de gel de silice 60. On obtient, après évaporation du solvant, le produit attendu sous forme d'une huile épaisse jaune pâle (1,4 g, rendement = 41%).

Spectre $^1H$ RMN ($CDCl_3$): spectre conforme à la formule
Spectre $^{29}Si$ RMN ($CDCl_3$): spectre conforme à la formule.

EXEMPLE 6

Préparation d'un composé de formule générale (I) dans laquelle R = B = $CH_3$, a = b = 6, et A représente un radical de formule (III) dans laquelle $R_1$ = $R_4$ = H, $R_3$ = tert-butyl, $R_2$ = $R_5$ = OH et Y = $-(CH_2)_3-$.

A une suspension de platine sur charbon à 5% (12 mg) dans du toluène sec (5 ml) à 90-100°C, sous azote et sous agitation, on ajoute goutte à goutte en 90 minutes, une solution toluénique (10 ml) d'allyl-2 tert-butyl-5 hydroquinone (1,45 g, 7 meq) et de polyméthylhydro-(45-50%)diméthylsiloxane copolymer (Petrarch Systems Inc., PS 122,5, 1,03 g, 6,32 meq en SiH) tout en maintenant la température entre 100 et 105°C. On laisse sous agitation et au reflux jusqu'à disparition des groupements SiH (absence de bande à 2180 $cm^{-1}$ en infrarouge), soit 8 heures. On filtre sur papier, élimine le solvant et lave deux fois à l'éthanol à 80%. L'huile jaune pâle obtenue est reprise dans le dichlorométhane. La phase organique est séchée sur sulfate de sodium et filtrée sur lit de gel de silice 60. On obtient, après évaporation du solvant, le produit attendu sous forme d'une huile épaisse jaune pâle (1,5 g, rendement = 64%).

Spectre $^1H$ RMN ($CDCl_3$): spectre conforme à la formule
Spectre $^{29}Si$ RMN ($CDCl_3$): spectre conforme à la formule.

EXEMPLE D'APPLICATION 1

LAIT CORPOREL (émulsion E/H)

| | | |
|---|---|---|
| - Composé de l'exemple 2 | | 0,2 g |
| - Copolymère PEG 45/dodécylglycol de formule : | | |
| $H\ (O\ CH\ CH_2)_x\ O\ (CH_2CH_2O)_y\ (CH_2-CHO)_z\ H$<br>avec $C_{10}H_{21}$ sur chacun des deux CH ramifiés (tel qu'imprimé sous le CH de chaque groupe) | | |
| où x = z = 11 et y = 45 | | |
| vendu sous la dénomination ELFACOS ST9 par AKZO | | 2,5 g |
| - Mélange de mono- et diester de l'acide oléique et de diglycérol, vendu sous la dénomination HOSTACERINE DGO par AMERICAN HOESCHT | | 2,5 g |
| - Montmorillonite modifiée par des groupements diméthyldialkyl(suif hydrogéné)ammonium, vendue sous la dénomination BENTONE 38 par NL INDUSTRIES | | 1,0 g |
| - Silicone volatile | | 8,0 g |
| - Huile de Purcellin | | 6,0 g |
| - Huile de Tournesol | | 6,0 g |
| - Conservateurs | | 0,35 g |
| - Glycérine | | 5,0 g |
| - Parfum | | 0,2 g |
| - Eau déminéralisée | qsp | 100,0 g |

On peut remplacer le composé de l'exemple 2 par la même quantité de composé de l'exemple 5.

EXEMPLE D'APPLICATION 2

FOND DE TEINT COMPACT

| | | |
|---|---|---|
| - Composé de l'exemple 3 | 0,15 | g |
| - Isostéarate d'isopropyle | 40,75 | g |
| - Huile d'amande douce | 8,0 | g |
| - Pigments | 42,0 | g |
| - Ozokérite | 3,0 | g |
| - Silice | 1,0 | g |
| - Cire de Carnauba | 5,0 | g |
| - Parahydroxybenzoate de propyle | 0,1 | g |

On peut remplacer le composé de l'exemple 3 par la même quantité de composé de l'exemple 6.

EXEMPLE D'APPLICATION 3

ROUGE A LEVRES

| | | |
|---|---|---|
| - Composé de l'exemple 1 | 0,4 | g |
| - Ozokérite | 16,0 | g |
| - Cire microcristalline | 6,0 | g |
| - Cire de Candellila | 9,0 | g |
| - Huile de sésame | 14,0 | g |
| - Huile de ricin | 6,0 | g |
| - Lanoline liquide | 8,0 | g |
| - Lanoline acétylée | 8,0 | g |
| - Talc | 5,0 | g |
| - Mica-titane | 10,0 | g |
| - DC - Red 7 Ca lake | 4,2 | g |
| - DC - Red 6 Ba lake | 2,3 | g |
| - FDC Yellow 5 | 0,8 | g |
| - Dioxyde de titane | 2,5 | g |
| - Huile de tournesol | 8,0 | g |

Mise en évidence du pouvoir anti-oxydant du polysiloxane dans le rouge à lèvres :

La base de rouge à lèvres est chauffée dans un bain thermostaté à 120°C, sous barbotage d'air à 30 ml/mn. Le courant gazeux est entraîné dans un second récipient contenant de l'eau. On suit la conductivité de ce milieu aqueux qui augmente avec l'apparition des produits secondaires d'oxydation (acides propionique, acétique, ...). Le temps d'induction mesuré correspond au temps de latence observé avant l'augmentation exponentielle de la conductivité.

Pour la base de rouge à lèvres sans anti-oxydant, le temps d'induction est de 79 minutes.

Pour la base de l'exemple d'application 3, le temps est de 490 minutes.

Comparativement, si l'on remplace le polysiloxane de l'exemple 1 par du ditert-butylhydroxy toluène (à

concentration équivalente en motif BHT pour le polysiloxane), soit 0,2% de BHT, le temps d'induction est de 225 minutes.

EXEMPLE D'APPLICATION 4

Mise en évidence du pouvoir antioxydant du composé de l'exemple 4, vis-à-vis de la vitamine F.

En opérant de la même façon que ci-dessus, mais en chauffant la vitamine F dans un bain thermostaté à 100°C au lieu de 120°C, on obtient les résultats suivants :

- vitamine F seule = temps d'induction : 20 minutes
- vitamine F + 0,34% de polysiloxane de l'exemple 4 = temps d'induction : 303 minutes.

## Revendications

### Revendications pour les Etats contractants suivants : AT, BE, CH, DE, FR, GB, IT, LI, NL, SE

1. Utilisation en cosmétique ou en dermatologie comme agent antioxydant de diorganopolysiloxanes choisis parmi ceux :

(i) de formule (I) :

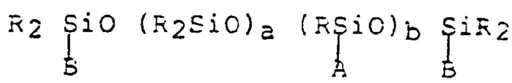

(I)

dans laquelle, les symboles :

R, identiques ou différents, sont choisis parmi les radicaux alkyles en $C_1$-$C_{10}$ ou phényle, au moins 80% des radicaux R étant méthyle,

B, identiques ou différents, sont choisis parmi les radicaux R et A,

a est un nombre entier choisi entre 0 et 200 inclus,

b est un nombre entier choisi entre 0 et 50 inclus, et si b est égal à 0, au moins l'un des deux symboles B est A,

A est un radical de formule :

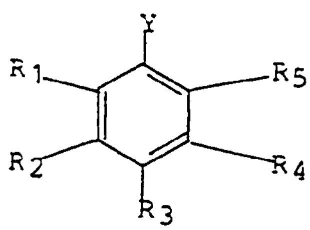

(III)

dans laquelle :

$R_1$ représente un atome d'hydrogène, un radical alkyle linéaire ou ramifié en $C_1$-$C_8$,

$R_2$ représente un atome d'hydrogène, un radical hydroxyle, un radical alkyle linéaire ou ramifié en $C_1$-$C_8$ ou un radical alcoxy linéaire ou ramifié en $C_1$-$C_8$,

$R_3$ représente un atome d'hydrogène, un radical hydroxyle ou un radical alkyle linéaire ou ramifié en $C_1$-$C_8$,

$R_4$ représente un atome d'hydrogène ou un radical alkyle linéaire ou ramifié en $C_1$-$C_8$,

$R_5$ représente un atome d'hydrogène, un radical hydroxyle ou un radical alcoxy linéaire ou ramifié en $C_1$-$C_8$,

étant entendu que l'un au moins des radicaux $R_2$, $R_3$ ou $R_5$ représente un radical hydroxyle, et que lorsque $R_2$ est alcoxy et $R_3$ est hydroxy, l'un au moins des radicaux $R_1$, $R_4$ ou $R_5$ est différent d'un atome d'hydrogène,

Y représente un radical divalent de formule :

$$(O)_n\text{-}CH_2\text{-}CH(R_6)\text{-}CH_2\text{-}$$

dans laquelle :

$R_6$ représente un atome d'hydrogène ou un radical alkyle en $C_1$-$C_4$ et n vaut 0 ou 1,

(ii) et ceux de formule (II) :

$$\left[(R_2\,SiO)_c\,(R\,\underset{A}{Si}O)_d\right] \qquad (II)$$

dans laquelle :

R et A ont les mêmes significations que celles indiquées dans la formule (I),
c est un nombre entier compris entre 1 et 20 inclus,
d est un nombre entier compris entre 2 et 20 inclus,
c + d est égal ou supérieur à 3.

**2.** Utilisation selon la revendication 1, caractérisé par le fait que les diorganopolysiloxanes sont des polymères statistiques ou à blocs de formule (I) ou (II) dans lesquelles :

- R est méthyle,
- B est méthyle,
- a est compris entre 5 et 20 inclus,
- b est compris entre 2 et 15 inclus,
- c + d est compris entre 3 et 10 inclus,
- dans le radical A, les alkyles sont choisis parmi les groupements méthyle, éthyle, n-propyle, n-butyle, tert-butyle, tétraméthyl-1,1,3,3 butyle, les radicaux alcoxy sont de préférence choisis parmi les méthoxy, et Y désigne $-(CH_2)_3-$ ou $-O(CH_2)_3-$.

**3.** Composition cosmétique ou dermatologique, caractérisée par le fait qu'elle contient dans un milieu cosmétiquement ou dermatologiquement acceptable, contenant une phase grasse d'origine animale, végétale ou synthétique et/ou une substance active susceptible de s'oxyder, au moins un diorganopolysiloxane de formule (I) ou (II) dans des concentrations comprises entre 0,01 et 5% et de préférence entre 0,05 et 3% en poids par rapport au poids total de la composition.

**4.** Composition selon la revendication 3, caractérisée par le fait qu'elle se présente sous forme de solutions huileuses, d'émulsions huile-dans-eau ou eau-dans-huile (crèmes ou laits), de bâtonnets solides, de lotions plus ou moins épaissies, de gels, de pommades, de tampons imbibés, de sprays aérosols ou mousse et de pains de savon.

**5.** Composition selon l'une quelconque des revendications 3 ou 4, caractérisée par le fait qu'elle contient en outre des agents adoucissants, des épaississants, des surgraissants, des émollients, des mouillants, des colorants, des agents tensio-actifs anioniques, cationiques, non ioniques, amphotères ou leurs mélanges, des filtres solaires et tout autre ingrédient habituellement utilisé dans des compositions cosmétiques ou dermatologiques.

**6.** Procédé de protection vis-à-vis de l'oxydation de substances cosmétiques ou dermatologiques oxydables, caractérisé par le fait que l'on incorpore auxdites substances un diorganopolysiloxane de formule (I) ou (II) définie dans les revendications 1 ou 2

**Revendications pour l'Etat contractant suivant : ES**

**1.** Procédé de préparation d'une composition cosmétique ou dermatologique stable, contenant dans un milieu cosmétiquement ou dermatologiquement acceptable, une phase grasse d'origine animale, végétale ou synthétique et/ou une substance active susceptible de s'oxyder, caractérisé par le fait que l'on introduit dans des concentrations comprises entre 0,01 et 5% en poids par rapport au poids total de la composition, comme agent antioxydant, un diorganopolysiloxane choisi parmi ceux :

(i) de formule (I) :

$$R_2\,\underset{B}{Si}O\,(R_2SiO)_a\,(R\underset{A}{Si}O)_b\,\underset{B}{Si}R_2 \qquad (I)$$

dans laquelle, les symboles :

R, identiques ou différents, sont choisis parmi les radicaux alkyles en $C_1$-$C_{10}$ ou phényle, au moins 80% des radicaux R étant méthyle,

B, identiques ou différents, sont choisis parmi les radicaux R et A,

a est un nombre entier choisi entre 0 et 200 inclus,

b est un nombre entier choisi entre 0 et 50 inclus, et si b est égal à 0, au moins l'un des deux symboles B est A,

A est un radical de formule :

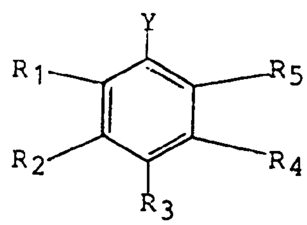

(III)

dans laquelle :

$R_1$ représente un atome d'hydrogène, un radical alkyle linéaire ou ramifié en $C_1$-$C_8$,

$R_2$ représente un atome d'hydrogène, un radical hydroxyle, un radical alkyle linéaire ou ramifié en $C_1$-$C_8$ ou un radical alcoxy linéaire ou ramifié en $C_1$-$C_8$,

$R_3$ représente un atome d'hydrogène, un radical hydroxyle ou un radical alkyle linéaire ou ramifié en $C_1$-$C_8$,

$R_4$ représente un atome d'hydrogène ou un radical alkyle linéaire ou ramifié en $C_1$-$C_8$,

$R_5$ représente un atome d'hydrogène, un radical hydroxyle ou un radical alcoxy linéaire ou ramifié en $C_1$-$C_8$,

étant entendu que l'un au moins des radicaux $R_2$, $R_3$ ou $R_5$ représente un radical hydroxyle, et que lorsque $R_2$ est alcoxy et $R_3$ est hydroxy, l'un au moins des radicaux $R_1$, $R_4$ ou $R_5$ est différent d'un atome d'hydrogène,

Y représente un radical divalent de formule :

$$(O)n\text{-}CH_2\text{-}CH(R_6)\text{-}CH_2\text{-}$$

dans laquelle :

$R_6$ représente un atome d'hydrogène ou un radical alkyle en $C_1$-$C_4$ et n vaut 0 ou 1,

(ii) et ceux de formule (II) :

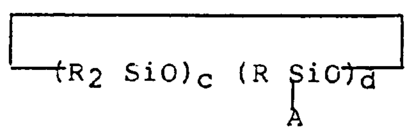

(II)

dans laquelle :

R et A ont les mêmes significations que celles indiquées dans la formule (I),

c est un nombre entier compris entre 1 et 20 inclus,

d est un nombre entier compris entre 2 et 20 inclus,

c + d est égal ou supérieur à 3.

**2.** Procédé selon la revendication 1, caractérisé par le fait que les diorganopolysiloxanes sont des polymères statistiques ou à blocs de formule (I) ou (II) dans lesquelles :

- R est méthyle,
- B est méthyle,
- a est compris entre 5 et 20 inclus,
- b est compris entre 2 et 15 inclus,
- c + d est compris entre 3 et 10 inclus,
- dans le radical A, les alkyles sont choisis parmi les groupements méthyle, éthyle, n-propyle, n-butyle, tert-butyle, tétraméthyl-1,1,3,3 butyle, les radicaux alcoxy sont de préférence choisis parmi les méthoxy, et Y désigne $-(CH_2)_3$- ou $-O(CH_2)_3$-.

3. Composition cosmétique caractérisée par le fait qu'elle contient dans un milieu cosmétiquement acceptable, contenant une phase grasse d'origine animale, végétale ou synthétique et/ou une substance active susceptible de s'oxyder, au moins un diorganopolysiloxane de formule (I) ou (II) dans des concentrations comprises entre 0,01 et 5% et de préférence entre 0,05 et 3% en poids par rapport au poids total de la composition.

4. Composition selon la revendication 3, caractérisé par le fait qu'elle se présente sous forme de solutions huileuses, d'émulsions huile-dans-eau ou eau-dans-huile (crèmes ou laits), de bâtonnets solides, de lotions plus ou moins épaissies, de gels, de pommades, de tampons imbibés, de sprays aérosol ou mousse ou de pains de savon.

5. Composition selon l'une quelconque des revendications 3 ou 4, caractérisée par le fait qu'elle contient en outre des agents adoucissants, des épaississants, des surgraissants, des émollients, des mouillants, des colorants, des agents tensio-actifs anioniques, cationiques, non ioniques, amphotères ou leurs mélanges, des filtres solaires et tout autre ingrédient habituellement utilisé dans des compositions cosmétiques.

6. Procédé de protection vis-à-vis de l'oxydation de substances cosmétiques ou dermatologiques oxydables, caractérisé par le fait que l'on incorpore auxdites substances un diorganopolysiloxane de formule (I) ou (II) définie dans les revendications 1 ou 2.

## Patentansprüche

### Patentansprüche für folgende Vertragsstaaten : AT, BE, CH, DE, FR, GB, IT, LI, NL, SE

1. Verwendung eines Antioxidationsmittels von Diorganopolysiloxanen in der Kosmetik oder Dermatologie, ausgewählt aus
   (1) aus Verbindungen der Formel (I):

$$R_2\,\underset{\substack{|\\B}}{Si}O\quad(R_2SiO)_a\quad(R\underset{\substack{|\\A}}{Si}O)_a\quad\underset{\substack{|\\B}}{Si}R_2 \qquad (I)$$

worin:

R gleich oder verschieden ist, und ausgewählt ist aus $C_1$-$C_{10}$-Alkylresten oder phenyl, wobei mindestens 80 % der Zahl der Reste R Methyl sind,

B gleich oder verschieden ist, und ausgewählt ist aus den Resten R oder Rest A,

a ist eine ganze Zahl zwischen 0 und einschließlich 200,

b ist eine ganze Zahl zwischen 0 und einschließlich 50, und wenn B gleich 0 ist, ist mindestens einer der beiden Symbole B gleich A,

A ist ein Rest der Formel

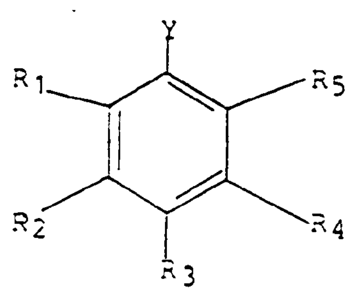

(III)

worin :

$R_1$ ein Wasserstoffatom, einen linearen oder verzweigten $C_1$-$C_8$-Alkylrest darstellt,

$R_2$ stellt ein Wasserstoffatom, einen Hydroxylrest, einen linearen oder verzweigten $C_1$-$C_8$-Alkylrest oder einen linearen oder verzweigten $C_1$-$C_8$-Alkoxyrest dar,

$R_3$ stellt ein Wasserstoffatom, einen Hydroxylrest oder einen linearen oder verzweigten $C_1$-$C_8$-

Alkylrest dar,

$R_4$ stellt ein Wasserstoffatom oder einen linearen oder verzweigten $C_1$-$C_8$-Alkylrest dar,

$R_5$ stellt ein Wasserstoffatom, einen Hydroxylrest oder einen linearen oder verzweigten $C_1$-$C_8$-Alkoxyrest dar,

mit der Maßgabe, daß mindestens einer der Reste $R_2$, $R_3$ oder $R_5$ ein Hydroxylrest ist, und daß, wenn $R_2$ Alkoxy und $R_3$ Hydroxy ist, mindestens einer der Reste $R_1$, $R_4$ oder $R_5$ von einem Wasserstoffatom verschieden ist,

Y stellt einen divalenten Rest dar:

$$(O)_n - CH_2 - CH(R_6) - CH_2$$

worin:

$R_6$ ein Wasserstoffatom oder einen $C_1$-$C_4$-Alkylrest darstellt und n 0 oder 1 ist,

(2) und aus Verbindungen der Formel (II):

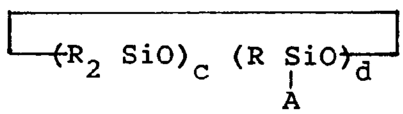 (II)

worin:

R und A dieselben Bedeutungen wie in Formel (I) haben,
c ist eine ganze Zahl zwischen 1 und einschließlich 20,
d ist eine ganze Zahl zwischen 2 und einschließlich 20,
c und d sind gleich oder größer 3.

2. Verwendung nach Anspruch 1, dadurch **gekennzeichnet**, daß die Diorganopolysiloxane statistische polymere oder Blockpolymere der Formel (I) oder (II) sind, worin:
   - R ist Methyl,
   - B ist Methyl,
   - a ist zwischen 5 und einschließlich 20,,
   - b ist zwischen 2 und einschließlich 15,
   - c + d sind zwischen 3 und einschließlich 10,
   - beim Rest A sind die Alkylreste ausgewählt aus Methyl, Ethyl, n-Propyl , n-Butyl, t-Butyl, 1,1,3,3,-Tetramethylbutyl, die Alkoxyreste sind bevorzugt Methoxy, und Y bedeutet einen $-(CH_2)_3$- oder $-O(CH_2)_3$-Rest.

3. Kosmetische oder dermatologische Zusammensetzung, dadurch **gekennzeichnet,** daß sie in einem kosmetischen oder dermatologisch annehmbaren Milieu mit einer Fettphase von tierischem, pflanzlichem oder synthetischen Ursprung und/oder einer oxydationsempfindlichen Substanz, mindestens ein Diorganopolysiloxan der Formel (I) oder (II) in den Konzentrationen zwischen 0,01 und 5 % und bevorzugt zwischen 0,05 und 3 Gew.-%, bezogen auf das Gesamtgewicht der Zusammensetzung, enthält.

4. Zusammensetzung nach Anspruch 3, dadurch **gekennzeichnet**, daß sie in Form von Öllösungen, Öl-in-Wasser- oder Wasser-in-Öl-Emulsionen (Creme oder Milch), festen Stäbchen, mehr oder weniger viskoser Lotionen, Gelen, Pomaden, imprägnierten Tampons, Aerosol- oder Cremesprays oder als Seifen vorliegt.

5. Zusammensetzung nach einem der Ansprüche 3 oder 4, dadurch **gekennzeichnet**, daß sie weitere Weichmacher, Verdickungsmittel, Auffettungsmittel, Erweichungsmittel, Befeuchtungsmittel, Farbstoffe, anionische, kationische, nichtionische, amphotere oberflächenaktive Mittel oder Mischungen davon, Lichtschutzfilter und andere Bestandteile, die üblicherweise in kosmetischen oder dermatologischen Zusammensetzungen verwendet werden, enthält.

6. Verfahren zum Schutz gegenüber Oxidation von kosmetischen oder dermatologisch oxidierbaren Substanzen, dadurch **gekennzeichnet,** daß man als Hilfssubstanzen ein Diorganopolysiloxan der Formel (I) oder (II) gemäß den Ansprüchen 1 oder 2 zusetzt.

**Patentansprüche für folgenden Vertragsstaat : ES**

1. Verfahren zur Herstellung einer kosmetischen oder dermatologischen Zusammensetzung, die in einem kosmetischen oder dermatologisch annehmbaren Milieu eine Fettsphase vom tierischen, pflanzlichen oder synthetischen Ursprung und/oder eine oxidationsempfindliche aktive Substanz enthält, dadurch **gekennzeichnet**, daß man in den Konzentrationen zwischen 0,01 und 5 Gew.-%, bezogen auf das Gesamtgewicht der Zusammensetzung, als Antioxidationsmittel ein Diorganopolysiloxan zugibt, ausgewählt aus

   (1) aus Verbindungen der Formel (I):

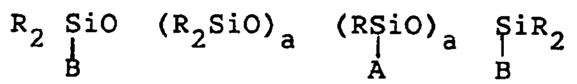

(I)

worin:

R gleich oder verschieden ist, und ausgewählt ist aus $C_1$-$C_{10}$-Alkylresten oder phenyl, wobei mindestens 80 % der Zahl der Reste R Methyl sind,

B gleich oder verschieden ist, und ausgewählt ist aus den Resten R oder Rest A,

a ist eine ganze Zahl zwischen 0 und einschließlich 200,

b ist eine ganze Zahl zwischen 0 und einschließlich 50, und wenn B gleich 0 ist, ist mindestens einer der beiden Symbole B gleich A,

A ist ein Rest der Formel

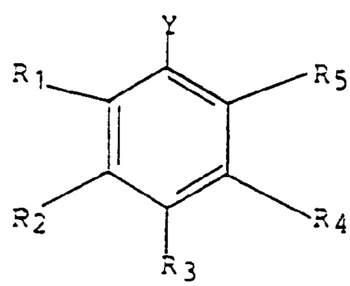

(III)

worin:

$R_1$ ein Wasserstoffatom, einen linearen oder verzweigten $C_1$-$C_8$-Alkylrest darstellt,

$R_2$ stellt ein Wasserstoffatom, einen Hydroxylrest, einen linearen oder verzweigten $C_1$-$C_8$-Alkylrest oder einen linearen oder verzweigten $C_1$-$C_8$-Alkoxyrest dar,

$R_3$ stellt ein Wasserstoffatom, einen Hydroxylrest oder einen linearen oder verzweigten $C_1$-$C_8$-Alkylrest dar,

$R_4$ stellt ein Wasserstoffatom oder einen linearen oder verzweigten $C_1$-$C_8$-Alkylrest dar,

$R_5$ stellt ein Wasserstoffatom, einen Hydroxylrest oder einen linearen oder verzweigten $C_1$-$C_8$-Alkoxyrest dar,

mit der Maßgabe, daß mindestens einer der Reste $R_2$, $R_3$ oder $R_5$ ein Hydroxylrest ist, und daß, wenn $R_2$ Alkoxy und $R_3$ Hydroxy ist, mindestens einer der Reste $R_1$, $R_4$ oder $R_5$ von einem Wasserstoffatom verschieden ist,

Y stellt einen divalenten Rest dar:

$$(O)_n - CH_2 - CH(R_6) - CH_2 -$$

worin:

$R_6$ ein Wasserstoffatom oder einen $C_1$-$C_4$-Alkylrest darstellt und n 0 oder 1 ist,

(2) und aus Verbindungen der Formel (II):

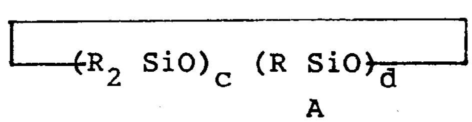

(II)

worin:

R und A dieselben Bedeutungen wie in Formel (I) haben,
c ist eine ganze Zahl zwischen 1 und einschließlich 20,
d ist eine ganze Zahl zwischen 2 und einschließlich 20,
c und d sind gleich oder größer 3.

**2.** Verfahren nach Anspruch 1, dadurch **gekennzeichnet**, daß die Diorganopolysiloxane statistische Polymere oder Blockpolymere der Formel (I) oder (II) sind, worin:
- R ist Methyl,
- B ist Methyl,
- a ist zwischen 5 und einschließlich 20,,
- b ist zwischen 2 und einschließlich 15,
- c + d sind zwischen 3 und einschließlich 10,
- beim Rest A sind die Alkylreste ausgewählt aus Methyl, Ethyl, n-Propyl , n-Butyl, t-Butyl, 1,1,3,3-Tetramethylbutyl, die Alkoxyreste sind bevorzugt Methoxy, und Y bedeutet einen $-(CH_2)_3-$ oder $-O(CH_2)_3-$ Rest.

**3.** Kosmetische Zusammensetzung, dadurch **gekennzeichnet**, daß sie in einem kosmetischen annehmbaren Milieu mit einer Fettphase von tierischem, pflanzlichem oder synthetischen Ursprung und/oder einer oxydationsempfindlichen Substanz, mindestens ein Diorganopolysiloxan der Formel (I) oder (II) in den Konzentrationen zwischen 0,01 und 5 % und bevorzugt zwischen 0,05 und 3 Gew.-%, bezogen auf das Gesamtgewicht der Zusammensetzung, enthält.

**4.** Zusammensetzung nach Anspruch 3, dadurch **gekennzeichnet**, daß sie in Form von Öllösungen, Öl-in-Wasser- oder Wasser-in-Öl-Emulsionen (Creme oder Milch), festen Stäbchen, mehr oder weniger viskosen Lotionen, Gelen, Pomaden, imprägnierten Tampons, Aerosol- oder Cremesprays oder als Seifen vorliegt.

**5.** Zusammensetzung nach einem der Ansprüche 3 oder 4, dadurch **gekennzeichnet**, daß sie weitere Weichmacher, Verdickungsmittel, Auffettungsmittel, Erweichungsmittel, Befeuchtungsmittel, Farbstoffe, anionische, kationische, nichtionische, amphotere oberflächenaktive Mittel oder Mischungen davon, Lichtschutzfilter und andere Bestandteile, die üblicherweise in kosmetischen Zusammensetzungen verwendet werden, enthält.

**6.** Verfahren zum Schutz gegenüber Oxidation von kosmetischen oder dermatologisch oxidierbaren Substanzen, dadurch **gekennzeichnet**, daß man als Hilfssubstanzen ein Diorganopolysiloxan der Formel (I) oder (II) gemäß den Ansprüchen 1 oder 2 zusetzt.

## Claims

### Claims for the following Contracting States : AT, BE, CH, DE, FR, GB, IT, LI, NL, SE

**1.** Use, as an antioxidizing agent in cosmetics or in dermatology, of diorganopolysiloxanes chosen from those:
(i) of formula (I) :

$$R_2\underset{\underset{B}{|}}{Si}O\ (R_2SiO)_a\ (R\underset{\underset{A}{|}}{Si}O)_b\ \underset{\underset{B}{|}}{Si}R_2 \qquad (I)$$

in which the symbols :
R, which are identical or different, are chosen from $C_1$-$C_{10}$ alkyl or phenyl radicals, at least 80 % of the number of the radicals R being methyl,
B, which are identical or different, are chosen from the radicals R and A,
a is an integer chosen between 0 and 200 inclusive,
b is an integer chosen between 0 and 50 inclusive,
and if b is equal to 0 at least one of the two symbols B is A,
A is a radical of formula :

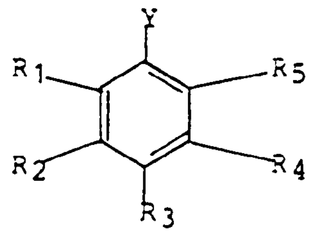

(III)

in which :

$R_1$ denotes a hydrogen atom or a $C_1$-$C_8$ linear or branched alkyl radical,

$R_2$ denotes a hydrogen atom, a hydroxy radical, a $C_1$-$C_8$ linear or branched alkyl radical or a $C_1$-$C_8$ linear or branched alkoxy radical,

$R_3$ denotes a hydrogen atom, a hydroxyl radical or a $C_1$-$C_8$ linear or branched alkyl radical,

$R_4$ denotes a hydrogen atom or a $C_1$-$C_8$ linear or branched alkyl radical,

$R_5$ denotes a hydrogen atom, a hydroxyl radical or a $C_1$-$C_8$ linear or branched alkoxy radical,

it being understood that at least on of the radicals $R_2$, $R_3$ and $R_5$ denotes a hydroxyl radical and that when $R_2$ is alkoxy and $R_3$ is hydroxy, at least one of the radicals $R_1$, $R_4$ and $R_5$ is other than a hydroxy atom,

Y denotes a divalent radical of formula :

$$(0)_n\text{-}CH_2\text{-}CH(R_6)\text{-}CH_2\text{-}$$

in which :

$R_6$ denotes a hydrogen atom or a $C_1$-$C_4$ alkyl radical and n has the value of 0 or 1,

(ii) and those of formula (II) :

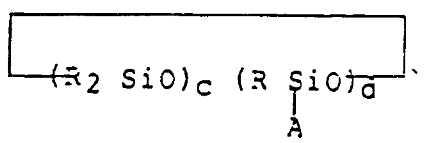

(II)

in which :

R and A have the same meanings as those shown in formula (I)

c in an integer between 1 and 20 inclusive,

d in an integer between 2 and 20 inclusive, and

c + d is equal to or greater than 3.

**2.** Use according to Claim 1, characterized in that the diorganopolysiloxanes are random or block polymers of formula (I) or (II), in which :

- R is methyl,
- B is methyl,
- a is between 5 and 20 inclusive,
- b is between 2 and 15 inclusive,
- c + d is between 3 and 10 inclusive, and
- in the radical A the alkyls are chosen from methyl, ethyl, n-propyl, n-butyl, tert-butyl and 1,1,3,3-tetramethylbutyl groups, the alkoxy radicals are preferably chosen from methoxy, and Y denotes $-(CH_2)_3$- or $-O(CH_2)_3$- .

**3.** Cosmetical or dermatological composition characterized in that it contains , in a cosmetically or dermatologically acceptable medium containing a fatty phase of animal, plant or synthetic origin, or an active substance susceptible to oxidation , at least one diorganopolysiloxane of formula (I) or (II) in concentrations of between 0.01 and 5 % and preferably between 0.05 and 3 % by weight relative to the total weight of the composition.

**4.** Composition according to claim 3, characterized in that it is presented in the form of oily solutions, of oil-in-water or water-in-oil emulsions (creams or milks), solid sticks, more or less thickened lotions, gels, pommades, satured pads , aerosol sprays or foam or soap tablets.

5. Composition according to either of Claims 3 and 4, characterized in that it additionally contains softening agents, thickeners ,superfattenings,emollients,wetting agents colorants, anionic,cationic, nonionic or amphoteric surface-active agents or mixtures thereof, sunscreens and any other ingredients usually employed in cosmetic or dermatological compositions.

6. Process for protecting oxidizable cosmetic or dermatological substances against oxidation, characterized in that one diorganopolys iloxane of formula (I) or (II) as defined in Claims 1 or 2 is incorporated in the said substances.

**Claims for the following Contracting State : ES**

1. Process for preparing a stable cosmetic or dermatological composition containing, in a cosmetically or dermatologically acceptable medium containing a fatty phase of animal, plant or synthetic origine, or an active susbtance susceptible to oxidation, characterized in that from 0.01 to 5 % by weight relative to the total weight of the composition of one diorganopolysiloxane chosen from those :
(i) of formula (I)

$$R_2\underset{B}{\underset{|}{Si}}O\ (R_2SiO)_a\ (R\underset{A}{\underset{|}{Si}}O)_b\ \underset{B}{\underset{|}{Si}}R_2 \qquad (I)$$

in which the symbols :

R, which are identical or different, are chosen from $C_1$-$C_{10}$ alkyl or phenyl radicals, at least 80 % of the number of the radicals R being methyl,

B, which are identical or different, are chosen from the radicals R and A,

a is an integer chosen between 0 and 200 inclusive,

b is an integer chosen between 0 and 50 inclusive,

and if b is equal to 0 at least one of the two symbols B is A,

A is a radical of formula :

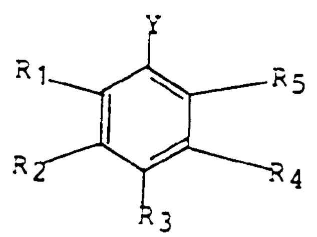

(III)

in which :

$R_1$ denotes a hydrogen atom or a $C_1$-$C_8$ linear or branched alkyl radical,

$R_2$ denotes a hydrogen atom, a hydroxy radical, a $C_1$-$C_8$ linear or branched alkyl radical or a $C_1$-$C_8$ linear or branched alkoxy radical,

$R_3$ denotes a hydrogen atom, a hydroxyl radical or a $C_1$-$C_8$ linear or branched alkyl radical,

$R_4$ denotes a hydrogen atom or a $C_1$-$C_8$ linear or branched alkyl radical,

$R_5$ denotes a hydrogen atom, a hydroxyl radical or a $C_1$-$C_8$ linear or branched alkoxy radical,

it being understood that at least one of the radicals $R_2$, $R_3$ and $R_5$ denotes a hydroxyl radical and that when $R_2$ is alkoxy and $R_3$ is hydroxy, at least one of the radicals $R_1$, $R_4$ and $R_5$ is other than a hydroxy atom,

Y denotes a divalent radical of formula :

$$(0)_n\text{-}CH_2\text{-}CH(R_6)\text{-}CH_2\text{-}$$

in which :

$R_6$ denotes a hydrogen atom or a $C_1$-$C_4$ alkyl radical and n has the value of 0 or 1,

(ii) and those of formula (II) :

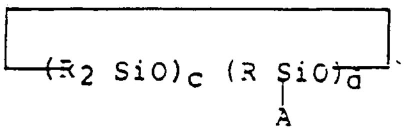

(II)

in which :

R and A have the same meanings as those shown in formula (I)

c in an integer between 1 and 20 inclusive,

d in an integer between 2 and 20 inclusive, and

c + d is equal to or greater than 3; is incorporated in the said composition as an antioxidizing agent.

2. Process according to Claim 1, characterized in that the diorganopolysiloxanes are random or block polymers of formula (I) or (II), in which :
   - R is methyl,
   - B is methyl,
   - a is between 5 and 20 inclusive,
   - b is between 2 and 15 inclusive,
   - c + d is between 3 and 10 inclusive, and
   - in the radical A the alkyls are chosen from methyl, ethyl, n-propyl, n-butyl, tert-butyl and 1,1,3,3-tetramethylbutyl groups, the alkoxy radicals are preferably chosen from methoxy, and Y denotes $-(CH_2)_3-$ or $-O(CH_2)_3-$ .

3. Cosmetical or dermatological composition characterized in that it contains , in a cosmetically or dermatologically acceptable medium containing a fatty phase of animal, plant or synthetic origin, or an active substance susceptible to oxidation , at least one diorganopolysiloxane of formula (I) or (II) in concentrations of between 0.01 and 5 % and preferably between 0.05 and 3 % by weight relative to the total weight of the composition.

4. Composition according to claim 3, characterized in that it is presented in the form of oily solutions, of oil-in-water or water-in-oil emulsions (creams or milks), solid sticks, more or less thickened lotions, gels, pommades, satured pads , aerosol sprays or foam or soap tablets.

5. Composition according to either of Claims 3 and 4, characterized in that it additionally contains softening agents, thickeners ,superfattenings,emollients,wetting agents colorants, anionic,cationic, nonionic or amphoteric surface-active agents or mixtures thereof, sunscreens and any other ingredients usually employed in cosmetic or dermatological compositions.

6. Process for protecting oxidizable cosmetic or dermatological substances against oxidation, characterized in that one diorganopolysiloxane of formula (I) or (II) as defined in Claims 1 or 2 is incorporated in the said substances.